# EUROPÄISCHE PATENTANMELDUNG

(11) **EP 1 153 597 A2**
(43) Veröffentlichungstag der Anmeldung: **14.11.2001**
(21) Anmeldenummer: 01103707.4
(22) Anmeldetag: 15.02.2001
(51) Int. Cl.: A61K 7/13

(54) **Mittel zum Färben von Keratinfasern enthaltend einen Assoziativverdicker**

(30) Priorität: 11.05.2000 DE 10023028
(71) Anmelder: Wella Aktiengesellschaft, 64274 Darmstadt (DE)
(72) Erfinder: Schmenger, Jürgen, 64331 Weiterstadt (DE); Abels, Wilhelm, Simi Valley, CA 93065 (US); Schmitt, Manfred, 64646 Heppenheim (DE)

(57) **Zusammenfassung**

Die vorliegende Erfindung betrifft oxidative beziehungsweise nicht-oxidative Färbemittel, welche in einem geeigneten kosmetischen Träger (a) Oxidationsfarbstoffvorstufen und/oder direktziehende Farbstoffe und (b) mindestens einen nichtionischen, amphiphilen Assoziatiwerdicker enthalten, sowie ein Verfahren zum Färben von Haaren unter Verwendung dieser Mittel.

## Beschreibung

Gegenstand der Erfindung sind Mittel zum Färben von Keratinfasern, insbesondere menschlichen Haaren, mit einem Gehalt an direktziehenden und/oder oxidativen Farbstoffen und bestimmten Assoziatiwerdickern, sowie ein Verfahren zum Färben von Haaren unter Verwendung dieser Mittel.

Übliche färbende Präparate liegen üblicherweise in Form von wässrigen -vorzugsweise verdickten- Lösungen oder Emulsionen vor und enthalten neben Farbstoffen beispielsweise Fettalkohole und/oder andere Ölkomponenten, Emulgatoren und Tenside, sowie gegebenenfalls Alkohole. Oxidationsfärbemittel bestehen in der Regel aus zwei Komponenten, (i) der die Farbstoffe enthaltenden Farbstoffträgermasse und (ii) der Oxidationsmittelzubereitung, die kurz vor dem Gebrauch miteinander vermischt und dann auf das zu färbende Haar aufgetragen werden. Je nach Viskosität und Mischungsverhältnis der beiden Komponenten ergibt sich beim Vermischen eine höhere oder niedrigere Viskosität. Eine gute Haftung des Färbemittels wird hierbei insbeondere durch eine höhere Viskosität des Färbemittels erzielt. Zudem benötigt der Friseur oft für seine Arbeiten höhere Viskositäten, beispielsweise bei speziellen Strähnen- oder Folientechniken sowie um gezielte Arbeiten mit dem Färbepinsel oder dem Akzentuierpinsel verrichten zu können. Dies erfordert jedoch bei den üblicherweise verwendeten Verdickungsmitteln eine deutliche Erhöhung der Menge an Verdickungsmittel und führt zu einer deutlichen Erhöhung der Formulierungskosten. Aufgrund der hohen Ausgangsviskositäten wird zudem eine homogene Vermischung der beiden Komponenten erschwert. Als Alternative hierzu bietet sich in bestimmten Fällen eine nachträgliche Erhöhung der Viskosität des Färbemittels an, wobei jedoch der Farbton durch die Viskositätseinstellung nicht verändert werden darf.

Eine nachträgliche Erhöhung der Viskosität des Färbemittels ohne Veränderung des Farbtons war jedoch bisher weder bei oxidativen noch bei nichtoxidativen Färbemitteln ohne weiteres möglich.

Es bestand daher ein grosses Bedürfnis nach einer kostengünstigen Verdickung der Farbstoffträgermasse, welche eine gute Vermischbarkeit der Farbstofflrägermasse mit dem Oxidationsmittel gewährleistet, zudem erforderlichenfalls eine einfache nachrägliche Erhöhung der Viskosität des gebrauchsfertigen Färbemittels ermöglicht und Färbemittel mit guten Haftungseigenschaften und Färbeeigenschaften ergibt.

Hierzu wurde nunmehr gefunden, dass sich bei Verwendung von bestimmten nichtionischen, amphiphilen Assoziatiwerdickern Mittel herstellen lassen, welche die an Färbemittel zu stellenden Anforderungen, insbesondere hinsichtlich Viskosität und Haftung, aber auch bezüglich der färberischen Eigenschaften in hervorragender Weise erfüllen.

Gegenstand der Erfindung sind daher Mittel zur Färbung von Keratinfasern, insbesondere Haaren, auf der Basis von Oxidationsfarbstoffvorstufen und/oder direktziehenden Farbstoffen, welche dadurch gekennzeichnet sind, dass sie in einem geeigneten kosmetischen Träger mindestens einen nichtionischen, amphiphilen Assoziativverdicker enthalten.

Der nichtionische, amphiphile Assoziatiwerdicker ist in der erfindungsgemäßen Zusammensetzung vorzugsweise in einer Menge von etwa 0,01 bis 20 Gewichtsprozent; insbesondere von etwa 0,1 bis 10 Gewichtsprozent enthalten.

Als nichtionischer, amphiphiler Assoziatiwerdicker wird ein Polymer eingesetzt, welches sowohl hydrophile als auch hydrophobe Gruppen enthält. Assoziatiwerdicker sind wasserlösliche Polymere und haben tensidartige hydrophobe Bestandteile, welche in der Lage sind, sich in einem hydrophilen, insbesondere wässrigen Medium sowohl mit sich selbst als auch mit anderen hydrophoben Stoffen zu assoziieren, das heisst in Wechselwirkung zu treten. Durch das daraus resultierende assoziative Netzwerk wird das Medium verdickt oder geliert. Typischerweise werden Assoziatiwerdicker durch Polymerisation von Polyethylenoxid-Prepolymeren und mindestens zweifach funktionellen, polykondensierbaren Stoffen wie zum Beispiel Isocyanaten hergestellt, wobei Monohydroxyverbindungen oder Dihydroxyverbindungen mit großen Aryl- Gruppen, Alkyl- Gruppen oder Aryl/Alkyl-Gruppen eingebaut werden, um die hydrophobe Modifikation bereitzustellen. Bevorzugte Assoziatiwerdicker sind daher hydrophob modifizierte Polyalkylenglykole. Hierbei wird der hydrophile Bestandteil durch Polyoxyalkyleneinheiten, vorzugwseise Polyoxyethyleneinheiten aber auch Polyoxypropyleneinheiten oder deren Gemisch gebildet. Der hydrophobe Bestandteil wird vorzugsweise aus Kohlenwasserstoffgruppen, beispielsweise langkettigen Alkylgruppen, Alkylarylgruppen oder Arylalkylgruppen gebildet. Besonders bevorzugte Assoziatiwerdicker sind hydrophob modifizierte Aminoplast-Polyether-Copolymere. Bezüglich deren Struktur und Herstellung wird auf die WO 96/40815 verwiesen. In der WO 96/40815 werden wasserdispergierbare oder wasserlösliche Copolymere beschrieben, welche die Reaktionsprodukte sind einer säurekatalysierten Polykondensation von mindestens zweifach funktionellen Aminoplastmonomeren und mindestens zweifach funktionellen Alkylenpolyethern sowie einfach funktionellen Verbindungen mit hydrophoben Gruppen. Geeignete Aminoplaste sind der Figur 1 der WO 96/40815 zu entnehmen. Besonders bevorzugt sind hierbei die Glycolurilderivate der Formel X der WO 96/40815. Geeignete Alkylenpolyether sind der Figur 2 der WO 96/40815 zu entnehmen. Bevorzugte Alkylenpolyether sind Polyethylenoxiddiole. Diese können einen Ethoxylierungsgrad von 20 bis 500, vorzugsweise 50 bis 350, besonders bevorzugt von 100 bis 250 haben. Geeignete einfach funktionelle Verbindungen mit hydrophoben Gruppen sind diejenigen der Formel XIV der WO 96/40815.

Erfindungsgemäß geeignete Assoziatiwerdicker sind vorzugsweise ausgewählt aus Polymeren der allgemeinen Formel (I) 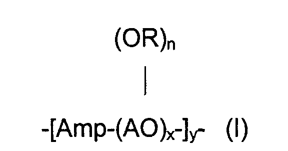 wobei Amp ein Aminoplastmonomer oder den Rest eines Aminoplastoligomers oder Aminoplastpolymers bedeutet, AO für eine Alkylenoxidgruppe steht, R für Wasserstoff, eine C1-C4-Alkylgruppe oder eine C1-C4-Acylgruppe steht und x und y Zahlen größer 1 sind.

Besonders bevorzugt sind die Reaktionsprodukte der säurekatalysierten Polykondensation von (a) Glykolurilen der allgemeinen Formel (II), 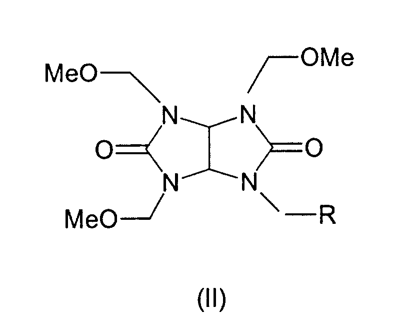 wobei R für H oder vorzugsweise für OMe steht, mit (b) Polyethylenoxiddiolen eines Ethoxylierungsgrades von 20 bis 500, vorzugsweise 50 bis 350, besonders bevorzugt von 100 bis 250 sowie (c) eines gegebenenfalls ethoxylierten hydrophoben Alkohols, Alkylphenols, Thiols, Carboxamids, Carbamats oder einer hydrophoben Carbonsäure, wie sie auf den Seiten 17 bis 19 der WO 96/40815 beschrieben sind. Ein besonders bevorzugtes Glykoluril ist das 1,3,4,6-Tetramethoxymethylglycoluril (TMMG).

Besonders geeignete Assoziatiwerdicker sind solche mit den INCI-Bezeichnungen Polyether-1, PEG-180/Octoxynol-40/TMMG Copolymer und PEG-180/Laureth-50/TMMG Copolymer und werden beispielsweise von der Firma Süd-Chemie AG, München/Deutschland unter den Handelsbezeichnungen Pure-Thix® HH, HL, L, M, TX-1442, TX-1450, TX-1451, TX-1452 und TX-1499 vertrieben.

Das erfindungsgemäße Färbemittel enthält vorzugsweise Oxidationsfarbstoffvorstufen, bei denen die Färbung unter Einwirkung von Oxidationsmitteln, wie zum Beispiel Wasserstoffperoxid, oder in Gegenwart von Luftsauerstoff erzeugt wird.

Als geeignete Oxidationsfarbstoffvorstufen können beispielsweise die folgenden Entwicklersubstanzen und Kupplersubstanzen und mit sich selbst kuppelnden Verbindungen genannt werden:
(i) Entwicklersubstanzen: 1,4-Diamino-benzol (p-Phenylendiamin), 1,4-Diamino-2-methyl-benzol (p-Toluylendiamin), 1,4-Diamino-2,6-dimethyl-benzol, 1,4-Diamino-3,5-diethyl-benzol, 1,4-Diamino-2,5-dimethyl-benzol, 1,4-Diamino-2,3-dimethyl-benzol, 2-Chlor-1,4-diaminobenzol, 1,4-Diamino-2-(thiophen-2-yl)benzol, 1,4-Diamino-2-(thiophen-3-yl)benzol, 1,4-Diamino-2-(pyridin-3-yl)benzol, 2,5-Diaminobiphenyl, 1,4-Diamino-2-methoxymethyl-benzol, 1,4-Diamino-2-aminomethyl-benzol, 1,4-Diamino-2-hydroxymethyl-benzol, 1,4-Diamino-2-(2-hydroxyethoxy)-benzol, 2-(2-(Acetylamino)ethoxy)-1,4-diamino-benzol, 4-Phenylamino-anilin, 4-Dimethylamino-anilin, 4-Diethylamino-anilin, 4-Dipropylamino-anilin, 4-[Ethyl(2-hydroxyethyl)amino]-anilin, 4-[Di(2-hydroxyethyl)amino]-anilin, 4-[Di(2-hydroxyethyl)amino]-2-methylanilin, 4-[(2-Methoxyethyl)amino]-anilin, 4-[(3-Hydroxypropyl)amino]-anilin, 4-[(2,3-Dihydroxypropyl)amino]-anilin, 1,4-Diamino-2-(2-hydroxyethyl)-benzol, 1,4-Diamino-2-(1-methylethyl)-benzol, 1,3-Bis[(4-aminophenyl)(2-hydroxyethyl)amino]-2-propanol, 1,4-Bis[(4-Aminophenyl)amino]-butan, 1,8-Bis(2,5-diaminophenoxy)-3,6-dioxaoctan, 4-Amino-phenol, 4-Amino-3-methyl-phenol, 4-Amino-3-(hydroxymethyl)-phenol, 4-Amino-3-fluorphenol, 4-Methylamino-phenol, 4-Amino-2-(aminomethyl)-phenol, 4-Amino-2-(hydroxymethyl)-phenol, 4-Amino-2-fluor-phenol, 4-Amino-2-[(2-hydroxyethyl)-amino]methyl-phenol, 4-Amino-2-methyl-phenol, 4-Amino-2-(methoxymethyl)-phenol, 4-Amino-2-(2-hydroxyethyl)-phenol, 5-Amino-salicylsäure, 2,5-Diamino-pyridin, 2,4,5,6-Tetraamino-pyrimidin, 2,5,6-Triamino-4-(1 H)-pyrimidon, 4,5-Diamino-1-(2-hydroxyethyl)-1H-pyrazol, 4,5-Diamino-1-(1-methylethyl)-1H-pyrazol, 4,5-Diamino-1-[(4-methylphenyl)methyl]-1H-pyrazol, 1-[(4-Chlorphenyl)methyl]-4,5-diamino-1H-pyrazol, 4,5-Diamino-1-methyl-1H-pyrazol, 2-Amino-phenol, 2-Amino-6-methyl-phenol, 2-Amino-5-methyl-phenol, allein oder im Gemisch miteinander.
(ii) Kupplersubstanzen: N-(3-Dimethylamino-phenyl)-harnstoff, 2,6-Diamino-pyridin, 2-Amino-4-[(2-hydroxyethyl)amino]-anisol, 2,4-Diamino-1-fluor-5-methyl-benzol, 2,4-Diamino-1-methoxy-5-methylbenzol, 2,4-Diamino-1-ethoxy-5-methyl-benzol, 2,4-Diamino-1-(2-hydroxyethoxy)-5-methyl-benzol, 2,4-Di[(2-hydroxyethyl)amino]-1,5-dimethoxybenzol, 2,3-Diamino-6-methoxy-pyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxyethoxy)-benzol, 1,3-Diamino-4-(2,3-dihydroxypropoxy)-benzol, 2,4-Diamino-1,5-di(2-hydroxyethoxy)-benzol, 1-(2-Aminoethoxy)-2,4-diamino-benzol, 2-Amino-1-(2-hydroxyethoxy)-4-methylamino-benzol, 2,4-Diaminophenoxy-essigsäure, 3-[Di(2-hydroxyethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Aminoethyl)-amino]-anilin, 1,3-Di(2,4-diaminophenoxy)-propan, Di(2,4-diaminophenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethylamino-phenol, 5-Amino-2-methyl-phenol, 5-Amino-4-fluor-2-methyl-phenol, 5-Amino-4-methoxy-2-methyl-phenol, 5-Amino-4-ethoxy-2-methyl-phenol, 3-Amino-2,4-dichlor-phenol, 5-Amino-2,4-dichlor-phenol, 3-Amino-2-methyl-phenol, 3-Amino-2-chlor-6-methyl-phenol, 3-Aminophenol, 2-[(3-Hydroxyphenyl)amino]-acetamid, 5-[(2-Hydroxyethyl)amino]-4-methoxy-2-methyl-phenol, 5-[(2-Hydroxyethyl)amino]-2-methyl-phenol, 3-[(2-Hydroxyethyl)amino]-phenol, 3-[(2-Methoxyethyl)amino]-phenol, 5-Amino-2-ethyl-phenol, 5-Amino-2-methoxy-phenol, 2-(4-Amino-2-hydroxyphenoxy)-ethanol, 5-[(3-Hydroxypropyl)amino]-2-methyl-phenol, 3-[(2,3-Dihydroxypropyl)amino]-2-methyl-phenol, 3-[(2-Hydroxyethyl)-amino]-2-methyl-phenol, 2-Amino-3-hydroxy-pyridin, 5-Amino-4-chlor-2-methyl-phenol, 1-Naphthol, 2-Methyl-1-naphthol, 1,5-Dihydroxynaphthalin, 1,7-Dihydroxy-naphthalin, 2,3-Dihydroxy-naphthalin, 2,7-Dihydroxy-naphthalin, 2-Methyl-1-naphthol-acetat, 1,3-Dihydroxybenzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxybenzol, 1,3-Dihydroxy-2-methyl-benzol, 3,4-Methylendioxy-phenol, 3,4-Methylendioxy-anilin, 5-[(2-Hydroxyethyl)amino]-1,3-benzodioxol, 6-Brom-1-hydroxy-3,4-methylendioxy-benzol, 3,4-Diamino-benzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxy-indol, 7-Hydroxy-indol, 2,3-Indolindion, allein oder im Gemisch miteinander.
(iii) Mit sich selbst kuppelnde Verbindungen: 2-Amino-5-methylphenol, 2-Amino-6-methylphenol, 2-Amino-5-ethoxyphenol oder 2-Propylamino-5-aminopyridin.

Die Gesamtmenge der in dem erfindungsgemäßen Mittel enthaltenen Oxidationsfarbstoffvorstufen beträgt etwa 0,01 bis 12 Gewichtsprozent, insbesondere etwa 0,2 bis 6 Gewichtsprozent.

Zur Erzielung bestimmter Farbnuancen können ferner auch übliche direktziehende Farbstoffe, beispielsweise Triphenylmethanfarbstoffe, aromatische Nitrofarbstoffe, Azofarbstofte, Chinonfarbstoffe, kationische oder anionische Farbstoffe in dem Färbemittel enthalten sein, beispielsweise 1,4-Bis[(2-hydroxyethyl)amino]-2-nitrobenzol, 1 -(2-Hydroxyethyl)amino-2-nitro-4-[di(2-hydroxyethyl)-amino]-benzol (HC Blue No. 2), 1-Amino-3-methyl-4-[(2-hydroxyethyl)-amino]-6-nitrobenzol (HC Violet No. 1), 4-[Ethyl-(2-hydroxyethyl)amino]-1-[(2-hydroxyethyl)amino]-2-nitrobenzol-hydrochlorid (HC Blue No. 12), 4-[Di(2-hydroxyethyl)amino]-1-[(2-methoxyethyl)amino]-2-nitrobenzol (HC Blue No. 11), 1-[(2,3-Dihydroxypropyl)amino]-4-[methyl-(2-hydroxyethyl)-amino]-2-nitrobenzol (HC Blue No. 10), 1-[(2,3-Dihydroxypropyl)amino]-4-[ethyl-(2-hydroxyethyl)amino]-2-nitrobenzol-hydrochlorid (HC Blue No. 9), 1-(3-Hydroxypropylamino)-4-[di(2-hydroxyethyl)amino]-2-nitrobenzol (HC Violet No. 2), 1-Methylamino-4-[methyl-(2,3-dihydroxypropyl)amino]-2-nitrobenzol (HC Blue No. 6), 2-((4-Amino-2-nitrophenyl)amino)-5-dimethylamino-benzoesäure (HC Blue No. 13), 1-Amino-4-[(2-hydroxyethyl)amino]-2-nitrobenzol (HC Red No. 7), 2-Amino-4,6-dinitro-phenol, 4-Amino-2-nitro-diphenylamin (HC Red No. 1), 1-Amino-4-[di(2-hydroxyethyl)amino]-2-nitrobenzol-hydrochlorid (HC Red No. 13), 1-Amino-5-chlor-4-[(2-hydroxyethyl)amino]-2-nitrobenzol, 4-Amino-1-[(2-hydroxyethyl)amino]-2-nitrobenzol (HC Red No. 3), 4-Amino-3-nitrophenol, 4-[(2-Hydroxyethyl)amino]-3-nitrophenol, 1-[(2-Aminoethyl)amino]-4-(2-hydroxyethoxy)-2-nitrobenzol (HC Orange No. 2), 4-(2,3-Dihydroxy-propoxy)-1-[(2-hydroxyethyl)amino]-2-nitrobenzol (HC Orange No. 3), 1-Amino-5-chlor-4-[(2,3-dihydroxypropyl)amino]-2-nitrobenzol (HC Red No. 10), 5-Chlor-1,4-[di(2,3-dihydroxypropyl)amino]-2-nitrobenzol (HC Red No. 11), 2-[(2-Hydroxyethyl)amino]-4,6-dinitrophenol, 4-Ethylamino-3-nitrobenzoesäure, 2-[(4-Amino-2-nitrophenyl)-amino]-benzoesäure, 2-Chlor-6-ethylamino-4-nitrophenol, 2-Amino-6-chlor-4-nitrophenol, 4-[(3-Hydroxypropyl)amino]-3-nitrophenol, 2,5-Diamino-6-nitropyridin, 1,2,3,4-Tetrahydro-6-nitrochinoxalin, 7-Amino-3,4-dihydro-6-nitro-2H-1,4-benzoxazin (HC Red No. 14), 1-Amino-2-[(2-hydroxyethyl)amino]-5-nitrobenzol (HC Yellow No. 5), 1-(2-Hydroxyethoxy)-2-[(2-hydroxyethyl)-amino]-5-nitrobenzol (HC Yellow No. 4), 1-[(2-Hydroxyethyl)amino]-2-nitrobenzol (HC Yellow No. 2), 2-[(2-Hydroxyethyl)-amino]-1-methoxy-5-nitrobenzol, 2-Amino-3-nitrophenol, 1-(2-Hydroxyethoxy)-3-methylamino-4-nitrobenzol, 2,3-(Dihydroxypropoxy)-3-methylamino-4-nitrobenzol, 2-[(2-Hydroxyethyl)amino]-5-nitrophenol (HC Yellow No. 11), 3-[(2-Aminoethyl)-amino]-1-methoxy-4-nitrobenzol-hydrochlorid (HC Yellow No.9), 1-[(2-Ureidoethyl)amino]-4-nitrobenzol, 4-[(2,3-Dihydroxypropyl)amino]-3-nitro-1-trifluormethyl-benzol (HC Yellow No. 6), 1-Chlor-2,4-bis[(2-hydroxyethyl)amino]-5-nitrobenzol (HC Yellow No. 10), 4-[(2-Hydroxy-ethyl)amino]-3-nitro-1-methylbenzol, 1-Chlor-4-[(2-hydroxyethyl)amino]-3-nitrobenzol (HC Yellow No. 12), 4-[(2-Hydroxyethyl)-amino]-3-nitro-1-trifluormethyl-benzol (HC Yellow No. 13), 4-[(2-Hydroxyethyl)amino]-3-nitro-benzonitril (HC Yellow No. 14), 4-[(2-Hydroxyethyl)-amino]-3-nitro-benzamid (HC Yellow No. 15), 1,4-Di[(2,3-dihydroxypropyl)amino]-9,10-anthrachinon, 1-[(2-Hydroxyethyl)amino]-4-methylamino-9,10-anthrachinon (Cl61505, Disperse Blue No. 3), 2-[(2-Aminoethyl)amino]-9,10-anthrachinon (HC Orange No. 5), 1-Hydroxy-4-[(4-methyl-2-sulfophenyl)amino]-9,10-anthrachinon, 1-[(3-Aminopropyl)-amino]-4-methylamino-9,10-anthrachinon (HC Blue No. 8),1-[(3-Aminopropyl)-amino]-9,10-anthrachinon (HC Red No. 8), 1,4-Diamino-2-methoxy-9,10-anthrachinon (Cl62015, Disperse Red No. 11, Solvent Violet No. 26), 1,4-Dihydroxy-5,8-bis[(2-hydroxyethyl)amino]-9,10-anthrachinon (Cl62500, Disperse Blue No. 7, Solvent Blue No. 69), 9-(Dimethylamino)-benzo[a]-phenoxazin-7-ium-chlorid (Cl51175; Basic Blue No. 6), Di[4-(diethyl-amino)phenyl][4-(ethylamino)naphthyl]-carbenium-chlorid (Cl42595; Basic Blue No. 7), 3,7-Di(dimethylamino)-phenothiazin-5-ium-chlorid (Cl52015; Basic Blue No. 9), Di[4-(dimethylamino)phenyl][4-(phenylamino)naphthyl]-carbenium-chlorid (Cl44045; Basic Blue No. 26), 2-[(4-(Ethyl(2-hydroxy-ethyl)amino)phenyl)azo]-6-methoxy-3-methyl-benzothiazolium-methylsulfat (Cl11154; Basic Blue No. 41), 8-Amino-2-brom-5-hydroxy-4-imino-6-[(3-(trimethylammonio)phenyl)-amino]-1(4H)-naphthalinon-chlorid (Cl56059; Basic Blue No. 99), Bis[4-(dimethylamino)phenyl][4-(methyl-amino)phenyl]carbenium-chlorid (Cl42535; Basic Violet No. 1), Tris[4-(dimethylamino)phenyl]carbeniumchlorid (Cl42555; Basic Violet No. 3), 2-[3,6-(Diethylamino)-dibenzopyranium-9-yl]-benzoesäure-chlorid (Cl45170; Basic Violet No. 10), Di(4-aminophenyl)(4-amino-3-methyl-phenyl)carbenium-chlorid (Cl42510; Basic Violet No. 14), 1,3-Bis[(2,4-diamino-5-methylphenyl)azo]-3-methylbenzol (Cl21010;Basic Brown No. 4), 1-[(4-Aminophenyl)azo]-7-(trimethylammonio)-2-naphthol-chlorid (Cl12250; Basic Brown No. 16), 1-[(4-Amino-2-nitrophenyl)azo]-7-(trimethylammonio)-2-naphthol-chlorid (Basic Brown No. 17), 1-[(4-Amino-3-nitrophenyl)azo]-7-(trimethylammonio)-2-naphthol-chlorid (Cl12251; Basic Brown No. 17), 3,7-Diamino-2,8-dimethyl-5-phenyl-phenazinium-chlorid (Cl50240; Basic Red No. 2), 1,4-Dimethyl-5-[(4-(dimethylamino)phenyl)-azo]-1,2,4-triazolium-chlorid (Cl11055; Basic Red No. 22), 2-Hydroxy-1-[(2-methoxyphenyl)azo]-7-(trimethylammonio)-naphthalin-chlorid (Cl12245; Basic Red No. 76), 2-[2-((2,4-Dimethoxy-phenyl)amino)ethenyl]-1,3,3-trimethyl-3H-indol-1-ium-chlorid (Cl48055; Basic Yellow No. 11), 3-Methyl-1-phenyl-4-[(3-(trimethylammonio)-phenyl)azo]-pyrazol-5-on-chlorid (Cl12719; Basic Yellow No. 57), Bis[4-(diethylamino)phenyl]phenylcarbeniumhydrogensulfat(1:1) (Cl42040; Basic Green No. 1), 1-[Di(2-hydroxyethyl)-amino]-3-methyl-4-[(4-nitro-phenyl)azo]-benzol (Cl11210, Disperse Red No. 17), 4-[(4-Aminophenyl)-azo]-1-[di(2-hydroxyethyl)amino]-3-methylbenzol (HC Yellow No. 7), 2,6-Diamino-3-[(pyridin-3-yl)azo]-pyridin, 6-Hydroxy-5-[(4-sulfophenyl)azo]-2-naphthalinsulfonsäure-dinatriumsalz (Cl15985; Food Yellow No. 3; FD&C Yellow No. 6), 2,4-Dinitro-1-naphthol-7-sulfonsäure-dinatriumsalz (Cl10316; Acid Yellow No. 1; Food Yellow No. 1), 2-(lndan-1,3-dion-2-yl)chinolin-x,x-sulfonsäure (Gemisch aus Mono- und Disulfonsäure) (Cl47005;D&C Yellow No. 10; Food Yellow No. 13; Acid Yellow No. 3), 5-Hydroxy-1-(4-sulfophenyl)-4-[(4-sulfophenyl)-azo]pyrazol-3-carbonsäure-trinatriumsalz (Cl19140; Food Yellow No. 4; Acid Yellow No. 23), 9-(2-Carboxyphenyl)-6-hydroxy-3H-xanthen-3-on (Cl45350; Acid Yellow No. 73; D&C Yellow No. 8), 5-[(2,4-Dinitrophenyl)-amino]-2-phenylamino-benzolsulfonsäure-natriumsalz (Cl10385; Acid Orange No. 3), 4-[(2,4-Dihydroxyphenyl)azo]-benzolsulfonsäuremononatriumsalz (Cl14270; Acid Orange No. 6), 4-[(2-Hydroxynaphth-1-yl)azo]-benzolsulfonsäure-natriumsalz (Cl15510; Acid Orange No. 7), 4-[(2,4-Dihydroxy-3-[(2,4-dimethylphenyl)azo]phenyl)azo]-benzolsulfonsäure-natriumsalz (Cl20170; Acid Orange No. 24), 4-Hydroxy-3-[(4-sulfonaphth-1-yl)azo]-1-naphthalin-sulfonsäure-dinatriumsalz (Cl14720; Acid Red No. 14), 6-Hydroxy-5-[(4-sulfonaphth-1-yl)azo]-2, 4-naphthalin-disulfonsäure-trinatriumsalz (Cl16255; Ponceau 4R; Acid Red No. 18), 3-Hydroxy-4-[(4-sulfonaphth-1-yl)azo]-2,7-naphthalin-disulfonsäure-trinatriumsalz (Cl16185; Acid Red No. 27), 8-Amino-1-hydroxy-2-(phenylazo)-3,6-naphthalin-disulfonsäure-dinatriumsalz (Cl17200; Acid Red No. 33), 5-(Acetylamino)-4-hydroxy-3-[(2-methylphenyl)azo]-2,7-naphthalin-disulfonsäure-dinatriumsalz (Cl18065; Acid Red No. 35), 2-(3-Hydroxy-2,4,5,7-tetraiod-dibenzopyran-6-on-9-yl)-benzoesäure-dinatriumsalz (Cl45430;Acid Red No. 51), N-[6-(Diethylamino)-9-(2,4-disulfophenyl)-3H-xanthen-3-yliden]-N-ethylethanammonium-hydroxid, inneres Salz, Natriumsalz (Cl45100; Acid Red No. 52), 8-[(4-(Phenylazo)-phenyl)azo]-7-naphthol-1,3-disulfonsäure-dinatriumsalz (Cl27290; Acid Red No. 73), 2',4',5',7'-Tetrabrom-3',6'-dihydroxyspiro-[isobenzofuran-1(3H),9'-[9H]xanthen]-3-on-dinatriumsalz (C145380; Acid Red No. 87), 2',4',5',7'-Tetrabrom-4,5,6,7-tetrachlor-3',6'-dihydroxyspiro[isobenzofuran-1(3H),9'[9H]xanthen]-3-on-dinatriumsalz (Cl45410; Acid Red No. 92), 3',6'-Dihydroxy-4',5'-diiodospiro[isobenzofuran-1(3H),9'(9H)-xanthen]-3-on-dinatriumsalz (C145425; Acid Red No. 95), (2-Sulfophenyl)di[4-(ethyl((4-sulfophenyl)methyl)amino)phenyl]-carbenium-dinatriumsalz, betain (C142090; Acid Blue No. 9; FD&C Blue No. 1), 1,4-Bis[(2-sulfo-4-methylphenyl)amino]-9,10-anthrachinon-dinatriumsalz (Cl 61570; Acid Green No. 25), Bis[4-(dimethylamino)phenyl]-(3,7-disulfo-2-hydroxy-naphth-1-yl)carbenium-inneres Salz, mononatriumsalz (Cl44090; Food Green No. 4; Acid Green No. 50), Bis[4-(diethylamino)phenyl](2,4-disulfophenyl)carbenium-inneres salz, Natriumsalz (2:1) (Cl42045; Food Blue No. 3; Acid Blue No. 1), Bis[4-(diethylamino)phenyl](5-hydroxy-2,4-disulfophenyl)carbenium-inneres salz, Calciumsalz (2:1) (Cl42051; Acid Blue No. 3), 1-Amino-4-(cyclohexylamino)-9,10-anthrachinon-2-sulfon-säure-natriumsalz (Cl62045; Acid Blue No. 62),2-(1,3-Dihydro-3-oxo-5-sulfo-2H-indol-2-yliden)-2,3-dihydro-3-oxo-1 H-indol-5-sulfonsäure-dinatriumsalz (Cl73015; Acid Blue No. 74), 9-(2-Carboxyphenyl)-3-[(2-methylphenyl)amino]-6-[(2-methyl-4-sulfophenyl)amino]xanthylium-inneres Salz, mononatriumsalz (Cl45190; Acid Violet No. 9), 1-Hydroxy-4-[(4-methyl-2-sulfophenyl)amino]-9,10-anthrachinon-natriumsalz (Cl60730; D&C Violett No. 2; Acid Violet No. 43), Bis[3-nitro-4-[(4-phenylamino)-3-sulfo-phenylamino]-phenyl]-sulfon (Cl10410; Acid Brown No. 13), 5-Amino-4-hydroxy-6-[(4-nitrophenyl)azo]-3-(phenylazo)-2,7-naphthalin-disulfonsäure-dinatriumsalz (Cl20470; Acid Black No. 1), 3-Hydroxy-4-[(2-hydroxynaphth-1-yl)azo]-7-nitro-1-naphthalin-sulfonsäure-chromkomplex (3:2) (Cl15711; Acid Black No. 52), 3-[(2,4-Dimethyl-5-sulfophenyl)azo]-4-hydroxy-1-naphthalin-sulfonsäure-dinatriumsalz (Cl14700; Food Red No. 1; FD&C Red No. 4), 4-(Acetylamino)-5-hydroxy-6-[(7-sulfo-4-[(4-sulfophenyl)azo]naphth-1-yl)azo]-1,7-naphthalin-disulfonsäure-tetranatriumsalz (Cl28440; Food Black No. 1) und 3-Hydroxy-4-(3-methyl-5-oxo-1-phenyl-4,5-dihydro-1H-pyrazol-4-ylazo)-naphthalin-1-sulfonsäure-natriumsalz, Chrom-Komplex (Acid Red No. 195), alleine oder in Kombination miteinander.

Die Gesamtmenge der direktziehenden Farbstoffe beträgt in dem erfindungsgemässen Mittel etwa 0,01 bis 7 Gewichtsprozent, vorzugsweise etwa 0,2 bis 4 Gewichtsprozent.

Weitere zur Haarfärbung bekannte und übliche Farbstoffe, die in dem erfindungsgemässen Färbemittel enthalten sein können, sind unter anderem in E. Sagarin, "Cosmetics, Science and Technology", Interscience Publishers Inc., New York (1957), Seiten 503 ff. sowie H. Janistyn, "Handbuch der Kosmetika und Riechstoffe", Band 3 (1973), Seiten 388 ff. und K. Schrader "Grundlagen und Rezepturen der Kosmetika", 2. Auflage (1989), Seiten 782-815 beschrieben.

Obwohl Oxidationsfärbemittel bevorzugt sind, ist es selbstverständlich ebenfalls möglich, dass das erfindungsgemässe Färbemittel in Form eines nicht-oxidativen Färbemittels auf Basis der vorstehend genannten direktziehenden Farbstoffen vorliegt.

Darüberhinaus können in dem erfindungsgemäßen Mittel Antioxidantien wie zum Beispiel Ascorbinsäure, Thioglykolsäure oder Natriumsulfit, sowie Komplexbildner für Schwermetalle, beispielsweise Ethylendiamino-tetraacetat oder Nitriloessigsäure, in einer Menge von bis zu etwa 0,5 Gewichtsprozent enthalten sein. Parfümöle können in der erfindungsgemäßen Farbträgermasse in einer Menge von bis zu etwa 1 Gewichtsprozent enthalten sein. Selbstverständlich kann das vorstehend beschriebene Haarfärbemittel gegebenenfalls weitere, für Haarfärbemittel übliche Zusätze, wie zum Beispiel Konservierungsstoffe und Parfümöle; Antioxidantien, beispielsweise Natriumsulfit, Thioglykolsäure oder Ascorbinsäure; Komplexbildner; Lösungsmittel wie Wasser, niedere aliphatische Alkohole, beispielsweise Ethanol, Propanol und Isopropanol, oder Glykole wie Glycerin und 1,2-Propylenglykol; Netzmittel oder Emulgatoren aus den Klassen der anionischen, kationischen, amphoteren oder nichtionogenen oberflächenaktiven Substanzen; Verdicker wie höhere Fettalkohole, Stärke oder Cellulosederivate; weiterhin Weichmacher; Vaseline; Silikonöle, Paraffinöl und Fettsäuren sowie außerdem Pflegestoffe, wie kationische Harze, Lanolinderivate, Cholesterin, Vitamine, Pantothensäure und Betain, enthalten. Die erwähnten Bestandteile werden in den für solche Zwecke üblichen Mengen verwendet, zum Beispiel die Netzmittel und Emulgatoren in Konzentrationen von 0,1 bis 30 Gewichtsprozent, die Verdicker in einer Menge von 0,1 bis 25 Gewichtsprozent und die Pflegestoffe in einer Konzentration von 0,1 bis 5,0 Gewichtsprozent. Besonders vorteilhaft ist hierbei der Zusatz von nicht-ionischen und/oder anionischen Tensiden oder Emulgatoren, wie zum Beispiel Fettalkoholsulfaten, insbesondere Laurylsulfat und Natriumcocoylsulfat, oxethylierten Fettalkoholsulfaten, insbesondere Natriumlaurylethersulfaten mit 2 bis 4 Ethylenoxideinheiten im Molekül, oxethylierten Fettsäureestern, oxethylierten Nonylphenolen, oxethylierten Fettalkoholen, Alkylbenzolsulfonaten oder Fettsäurealkanolamiden, in einer Gesamtmenge von etwa 0,1 bis 30 Gewichtsprozent, vorzugsweise 0,2 bis 15 Gewichtsprozent.

Der pH-Wert des erfindungsgemäßen Färbemittels liegt bei nicht-oxidativen Färbemitteln auf der Basis von direktziehenden Farbstoffen im Bereich von etwa 5 bis 10, vorzugsweise 6 bis 9, während bei oxidativen Färbemitteln auf der Basis von Oxidationsfarbstoffvorstufen der pH-Wert in einem Bereich von etwa 6 bis 12, vorzugsweise 9 bis 11, liegt, wobei der pH-Wert des gebrauchsfertigen Oxidationshaarfärbemittels (das heißt der Mischung des erfindungsgemäßen Haarfärbemittels mit dem Oxidationsmittel) etwa 5,5 bis 10, vorzugsweise 6 bis 9, beträgt.

Je nach Zusammensetzung und gewünschtem pH-Wert des Färbemittels erfolgt die Einstellung des pH-Wertes vorzugsweise mit Ammoniak oder organischen Aminen, wie zum Beispiel Glucaminen, Aminomethylpropanol, Monoethanolamin oder Triethanolamin, anorganischen Basen, beispielsweise Natriumhydroxid, Kaliumhydroxid, Natriumcarbonat oder Calciumhydroxid, beziehungsweise organischen oder anorganischen Säuren, wie zum Beispiel Milchsäure, Zitronensäure, Essigsäure oder Phosphorsäure.

Das erfindungsgemäße Mittel wird vorzugsweise in Form einer wässrigen oder wässrig-alkoholischen Zubereitung, beispielsweise als verdickte Lösung, als Emulsion, als Creme oder als Gel, konfektioniert. Als Alkohole können insbesondere die für kosmetische Zwecke üblicherweise verwendeten niederen Alkohole mit 1 bis 4 Kohlenstoffatomen wie zum Beispiel Ethanol und Isopropanol enthalten sein.

Für die Anwendung zur oxidativen Färbung vermischt man das vorstehend beschriebene Färbemittel unmittelbar vor dem Gebrauch mit einem Oxidationsmittel (welches ebenfalls mit einem nichtionischen, amphiphilen Assoziatiwerdicker verdickt ist) und trägt eine für die Färbung ausreichende Menge, in der Regel etwa 60 bis 200 Gramm, der gebrauchsfertigen Zubereitung auf die Faser auf.

Sofern das erfindungsgemäße Färbemittel keine Oxidationsfarbstoffvorstufen enthält beziehungsweise Oxidationsfarbstoffvorstufen enthält, welche mit Luftsauerstoff leicht oxidierbar sind, kann es ohne vorheriges Vermischen mit einem Oxidationsmittel direkt auf die Keratinfaser aufgetragen werden.

Als Oxidationsmittel zur Entwicklung der Färbung kommen hauptsächlich Wasserstoffperoxid oder dessen Additionsverbindungen an Harnstoff, Melamin oder Natriumborat in Form einer 1- bis 12prozentigen, vorzugsweise 1,5- bis 6prozentigen wässrigen Lösung in Betracht. Das Mischungsverhältnis von Färbemittel zu Oxidationsmittel ist abhängig von der Konzentration des Oxidationsmittels und beträgt in der Regel etwa 5:1 bis 1:2, vorzugsweise 1:1, wobei der Gehalt an Oxidationsmittel in der gebrauchsfertigen Zubereitung vorzugsweise etwa 0,5 bis 8 Gewichtsprozent, insbesondere 1 bis 4 Gewichtsprozent, beträgt.

Man läßt das gebrauchsfertige Färbemittel bei 15 bis 50 °C etwa 10 bis 45 Minuten, vorzugsweise etwa 15 bis 30 Minuten lang, auf die Keratinfaser (zum Beispiel menschliche Haare) einwirken, spült sodann die Faser mit Wasser aus und trocknet sie. Gegebenenfalls wird im Anschluß an diese Spülung mit einem Shampoo gewaschen und eventuell mit einer schwachen organischen Säure, wie zum Beispiel Weinsäure, nachgespült. Anschließend wird die Keratinfaser getrocknet.

Es zeigte sich, dass bei einem erfindungsgemässen Oxidationsfärbemittel auch noch nach dem Vermischen mit dem Oxidationsmittel die Viskosität nach den Wünschen des Anwenders nachträglich ohne weiteres erhöht werden kann, wodurch einfachere und kostengünstigere Basisformulierungen möglich sind. Ein mit der erfindungsgemäßen Zusammensetzung hergestelltes Färbemittel erfüllt die in Bezug auf die Hafteigenschaften, Auftrageverhalten und Viskositätseinstellung gestellten Anforderungen in bester Weise, zeigt ein verbessertes Fließverhalten bei der Anwendung und ist spürbar leichter aufzutragen.

Die nachfolgenden Beispiele sollen den Gegenstand der Erfindung näher erläutern ohne diesen hierauf zu beschränken.

### Beispiele

### Beispiel 1: Farbverdicker zur nachträglichen Einstellung der Viskosität von Färbemitteln

| | |
|---|---|
| 30,0 g | PEG-180/Laureth-50/TMMG Copolymer, 20 %ige wässrige Lösung (Pure Thix® M) |
| 0,2 g | PHB Methylester |
| 1,8 g | Propylenglycol |
| ad 100,0 g | Wasser, vollentsalzt |

### Beispiel 2: Farbverdicker zur nachträglichen Einstellung der Viskosität von Färbemitteln mit zusätzlicher Pflege

| | |
|---|---|
| 1,3 g | Polyether-1, 20 %ige wässrige Lösung (Pure Thix® HH ) |
| 0,2 g | PHB Metylester |
| 1,8 g | Propylenglycol |
| 0,6 g | Dimethicone Propyl PG Betaine |
| 1,0 g | Dimethicone Copolyol |
| ad 100,0 g | Wasser, vollentsalzt |

### Beispiel 3: Oxidationshaarfärbemittel

### Komponente (A): Farbträgermasse

| | |
|---|---|
| 2,0 g | PEG-180/Laureth-50/TMMG Copolymer (Pure Thix® TX-1450) |
| 3,0 g | Natriumlaurylalkohol-diglykolethersulfat, 28 %ige wässrige Lösung |
| 2,8 g | 2,5-Diaminotoluolsulfat |
| 1,0 g | Resorcin |
| 0,4 g | m-Aminophenol |
| 0,2 g | 2-Amino-4-(2'-hydroxyethylamino)-anisolsulfat |
| 0,3 g | Ascorbinsäure |
| 0,1 g | Ethylendiamintetraessigsäure |
| 12,2 g | Ammoniak, 25 %ige wässrige Lösung |
| 2,0 g | Ethanol |
| ad 100,0 g | Wasser |

### Komponente (B): Wasserstoffperoxid - Emulsion

| | |
|---|---|
| 10,0 g | Cetylstearylalkohol |
| 1,5 g | Cholesterin |
| 4,0 g | Natriumlauryalkohol-diglykolethersulfat, 28%ige wässrige Lösung |
| 17,0 g | Wasserstoffperoxid, 35 %ige wässrige Lösung |
| 0,3 g | Parfüm |
| ad 100,0 g | Wasser |

Man vermischt vor dem Gebrauch 40 g der flüssigen Farbträgermasse (A) mit 80 g der Wasserstoffperoxid-Emulsion (B), entsprechend einem Mischungsverhältnis von (A):(B) von 1:2, und trägt 120 g dieses Gemisches auf graues, menschliches Haar auf. Nach einer Einwirkungszeit von 20 Minuten bei Raumtemperatur wird das Haar mit Wasser ausgespült getrocknet. Das so behandelte Haar hat einen gleichmäßigen, dunkelbraunen Ton angenommen. Das erfindungsgemäße Mittel haftet sehr gut auf dem Haar ohne abzulaufen.

### Beispiel 4: Gelförmiges Oxidationshaarfärbemittel zur Hellerfärbung

### Komponente (A): Flüssige Farbträgermasse

| | |
|---|---|
| 3,00 g | PEG-180/Octoxynol-40/TMMG Copolymer |
| 6,00 g | Nonylphenol oxethyliert mit 4 Mol Ethylenoxid |
| 6,00 g | Ölsäure |
| 0,50 g | para-Phenylendiamin |
| 0,07 g | Resorcin |
| 5,00 g | Natriumlaurylalkohol-diglykolethersulfat, 28%ige wässrige Lösung |
| 1,00 g | Ethylendiamintetraessigsäure-Dinatriumsalz |
| 18,00 g | Ammoniak, 25 %ige wässrige Lösung |
| 8,00 g | Ethanol |
| ad 100 g | Wasser |

### Komponente (B): Wasserstoffperoxid-Emulsion

| | |
|---|---|
| 10,0 g | Cetylstearylalkohol |
| 1,5 g | Cholesterin |
| 4,0 g | Natriumlaurylalkohol-diglykolethersulfat, 28 %ige wässrige Lösung |
| 35,0 g | Wasserstoffperoxid, 35 %ige wässrige Lösung |
| 0,3 g | Parfüm |
| ad 100,0 g | Wasser |

Man vermischt vor dem Gebrauch 40 g der flüssigen Farbträgermasse (A) mit 80 g der Wasserstoffperoxid-Emulsion (B), entsprechend einem Mischungsverhältnis von (A):(B) von 1:2, und trägt 120 g dieses Gemisches auf graues, menschliches Haar auf. Nach einer Einwirkungszeit von 20 Minuten bei Raumtemperatur wird das Haar mit Wasser ausgespült getrocknet. Das so behandelte Haar ist vom Haaransatz bis zu den Haarspitzen gleichmäßig hellbraun gefärbt. Das erfindungsgemäße Mittel haftet sehr gut auf dem Haar ohne abzulaufen.

### Beispiel 5: Cremeförmiges Oxidationshaarfärbemittel zur Hellerfärbung

| | |
|---|---|
| 3,00 g | PEG-180/Laureth-50/TMMG Copolymer (Pure Thix® TX-1450) |
| 15,00 g | Cetylalkohol |
| 3,50 g | Natriumlaurylalkohol-diglykolethersulfat (28prozentige wäßrige Lösung) |
| 3,00 g | Monoethanolamin |
| 1,30 g | 1-Methyl-2,5-diaminobenzol |
| 1,00 g | Bienenwachs |
| 0,65 g | Resorcin |
| 0,50 g | Keratinhydrolysat |
| 0,50 g | Seidenproteinhydrolysat |
| 0,50 g | 2-Amino-6-chlor-4-nitrophenol |
| 0,30 g | Ascorbinsäure |
| ad 100,00 g | Wasser |

50 g des vorstehenden Haarfärbemittels werden unmittelbar vor Gebrauch mit 50 g einer 2prozentigen wässrigen Wasserstoffperoxidlösung vermischt. Das erhaltene Gemisch wird anschließend auf blonde Naturhaare aufgetragen. Nach einer Einwirkungszeit von 30 Minuten bei 40 °C wird das Haar mit Wasser gespült und getrocknet. Es wird ein gleichmäßiger, kräftiger, leuchtender goldorangefarbener Farbton erhalten.

### Beispiel 6: (nicht-oxidative) Haartönung

| | |
|---|---|
| 1,8 g | PEG-180/Laureth-50/TMMG Copolymer (Pure Thix® TX-1450) |
| 5,0 g | Natriumlaurylsulfat |
| 1,5 g | 2-Amino-6-chlor-4-nitrophenol |
| 1,0 g | Monoethanolamin |
| 1,0 g | Bienenwachs |
| 0,5 g | Keratinhydrolysat |
| 0,3 g | Seidenproteinhydrolysat |
| 0,2 g | Glycin |
| ad 100,0 g | Wasser |

Das Haarfärbemittel wird gleichmäßig auf das Haar aufgetragen. Aufgrund seiner hervorragenden Viskositätseigenschaften haftet das Mittel sehr gut auf dem Haar. Nach einer Einwirkungszeit von 20 Minuten bei 20 °C wird das Haar mit lauwarmem Wasser ausgespült, zur Frisur gelegt und getrocknet. Das so behandelte Haar weist eine gleichmäßige, kräftig leuchtende goldorange Färbung auf.

Alle in der vorliegenden Anmeldung genannten Prozentangeaben stellen, soweit nicht anders angegeben, Gewichtsprozente dar.

## Patentansprüche

1. Mittel zur Färbung von Keratinfasern auf der Basis von Oxidationsfarbstoffvorstufen und/oder direktziehenden Farbstoffen, **dadurch gekennzeichnet, dass** es in einem geeigneten kosmetischen Träger mindestens einen nichtionischen, amphiphilen Assoziatiwerdicker enthält.

2. Mittel nach Anspruch 1, **dadurch gekennzeichnet, dass** der nichtionische, amphiphile Assoziatiwerdicker ausgewählt ist aus hydrophob modifizierten Polyalkylenglykolen.

3. Mittel nach Anspruch 1, **dadurch gekennzeichnet, dass** der nichtionische, amphiphile Assoziatiwerdicker ausgewählt ist aus hydrophob modifizierten Aminoplast-Polyether-Copolymeren.

4. Mittel nach Anspruch 1, **dadurch gekennzeichnet, dass** der nichtionische, amphiphile Assoziatiwerdicker ausgewählt ist aus Polymeren der allgemeinen Formel (I) 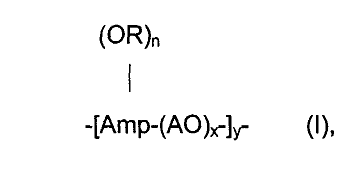 wobei Amp ein Aminoplastmonomer oder den Rest eines Aminoplastoligomers oder Aminoplastpolymers bedeutet, AO für eine Alkylenoxidgruppe steht, R für Wasserstoff, eine C1-C4-Alkylgruppe oder eine C1-C4-Acylgruppe steht und x und y Zahlen größer 1 sind.

5. Mittel nach Anspruch 1, **dadurch gekennzeichnet, dass** der nichtionische, amphiphile Assoziativverdicker ausgewählt ist aus Reaktionsprodukten der säurekatalysierten Polykondensation von (a) Glykolurilen der allgemeinen Formel (II), 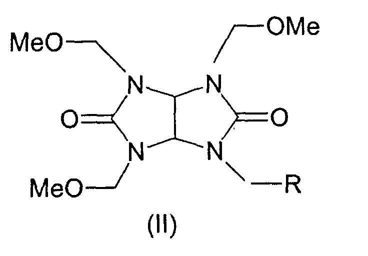 wobei R für H oder vorzugsweise für OMe steht, mit (b) Polyethylenoxiddiolen eines Ethoxylierungsgrades von 20 bis 500, sowie (c) eines gegebenenfalls ethoxylierten hydrophoben Alkohols, Alkylphenols, Thiols, Carboxamids, Carbamats oder einer hydrophoben Carbonsäure. Ein besonders bevorzugtes.

6. Mittel nach Anspruch 5, dadurch gekenn-zeichnet, dass das Glykoluril der Formel (II) das 1,3,4,6-Tetramethoxy-methylglycoluril ist.

7. Mittel nach einem der Ansprüche 1 bis 6, **dadurch gekennzeichnet, dass** der nichtionische, amphiphile Assoziativ-verdicker ausgewählt ist aus Polyether-1, PEG-180/Octoxynol-40/TMMG Copolymer und PEG-180/Laureth-50/TMMG Copolymer.

8. Mittel nach einem der Ansprüche 1 bis 7, **dadurch gekennzeichnet, dass** es 0,01 bis 12 Gewichtsprozent Oxidationsvorstufen enthält und vor der Anwendung mit einem Oxidationsmittel vermischt wird.

9. Mittel nach einem der Ansprüche 1 bis 7, **dadurch gekennzeichnet, dass** es 0,01 bis 12 Gewichtsprozent Oxidationsvorstufen enthält, welche durch Luftsauerstoff oxidierbar sind.

10. Verfahren zum Färben von Keratinfasern, bei dem man ein Mittel nach einem der Ansprüche 1 bis 9 auf die Keratinfaser aufträgt und nach einer Einwirkungszeit von 10 bis 45 Minuten bei 15 bis 50 °C die Faser mit Wasser ausspült und trocknet.
